Europäisches Patentamt

European Patent Office

Office européen des brevets

(11) Publication number: **0 404 576**
**A2**

# (12) EUROPEAN PATENT APPLICATION

(21) Application number: **90306806.2**

(22) Date of filing: **21.06.90**

(51) Int. Cl.5: **C12N 15/86, C12N 15/39, C12N 15/45, A61K 39/17, A61K 39/275**

(30) Priority: **22.06.89 JP 160157/89**

(43) Date of publication of application:
**27.12.90 Bulletin 90/52**

(84) Designated Contracting States:
**AT BE CH DE DK ES FR GB GR IT LI LU NL SE**

(71) Applicant: **NIPPON ZEON CO., LTD.**
**6-1, 2-chome, Marunouchi, Chiyoda-ku**
**Tokyo(JP)**

Applicant: **SHIONOGI & CO., LTD.**
**1-8, Doshomachi 3-chome Chuo-ku**
**Osaka 541(JP)**

(72) Inventor: **Yangida, Noboru**
**1640 South Shore Drive**
**A-1 East Lansing, Michigan 48823(US)**
Inventor: **Saeki, Sakiko**
**16-8 Higashiyaguchi, 1-chome, Ota-ku**
**Tokyo(JP)**
Inventor: **Okawa, Setsuko**
**17-13 Shinohara Nishimachi, Kohoku-ku**
**Yokohama-shi(JP)**
Inventor: **Kamogawa, Kouichi**
**2153-42 Kamariyacho, Kanazawa-ku**
**Kanagawa-ken(JP)**
Inventor: **Iritani, Yoshikazu**
**151 Fukakusa Okamedani Manjojikicho,**
**Fushimi-ku**
**Kyoto-shi(JP)**
Inventor: **Sawaguchi, Kazunari**
**789-21 Morishiri, Konancho, Koka-gun**
**Shiga-ken(JP)**

(74) Representative: **Harrison, David Christopher**
**et al**
**MEWBURN ELLIS 2 Cursitor Street**
**London EC4A 1BQ(GB)**

(54) **Recombinant avipoxvirus.**

(57) A recombinant Avipoxvirus having inserted all or a part of cDNA coding for Newcastle disease virus-derived fused protein in a genome region non-essential to proliferation of Avipoxvirus and a vaccine for fowl comprising the recombinant Avipoxvirus.

## RECOMBINANT AVIPOXVIRUS

The present invention relates to a recombinant Avipoxvirus and more particularly, to a recombinant Avipoxvirus having inserted cDNA derived from Newcastle disease virus into a DNA region non-essential to proliferation of Avipoxvirus.

In recent years, a method of constructing recombinant vaccinia virus having inserted exogenous DNA into vaccinia virus has been devised and there has been come to propose a method utilizing recombinant vaccinia virus obtained as live vaccine using, as exogenous DNA, for example, DNA coding for the antigen of infectious diseases (for example, Published Unexamined Japanese Patent Application KOKAI No. 129971/83, Public Disclosure Nos. 500518/85, 501957/86, etc.). According to this method, it is possible to insert a variety of exogenous DNAs depending upon purpose and, the method is expected to be promising as a new process for producing live vaccine.

In vaccinia virus, however, its host region is limited. For this reason, it is almost impossible to apply techniques of recombinant vaccinia virus to production of, for example, avian live vaccine and for the purpose of producing avian live vaccine, a suggested promising method is to insert exogenous DNA into Fowlpoxvirus in lieu of vaccinia virus (Avian Diseases, vol. 30, No. 1, 24-27). Based on such a concept, processes for producing recombinant Avipoxvirus have been developed recently (Virus Research, vol. 10, 343-356 (1988), Vaccine, vol. 6, 504-508 (1988)).

Further with respect to Newcastle disease virus (hereafter referred to as NDV), cDNA coding for hemagglutinin neuraminidase (hereafter referred to as HN) and membrane fused protein (hereafter referred to as F protein) has recently been found [for example, J. Gen. Virol., 67, 1917-1927 (1986), Published Unexamined Japanese Patent Application KOKAI No. 163693/87]. However, most viruses, especially RNA virus such as NDV, tend to undergo mutation and a danger that vaccine-ineffective mutant virus might emerge is not ignorable. Particularly in recombinant live vaccine in which a single antigenic gene alone has been inserted, it is highly likely that vaccine-ineffective mutant virus might appear, as compared to the existing vaccine. For this reason, it has been attempted to introduce F protein gene (hereafter referred to as F gene) having higher preservation property in viral strains as antigenic gene than in HN [Virus Res., 7, 241-255 (1987), ibid., 8, 217-232 (1988), ibid., 5, 343-356 (1986), J. Gen. Virol., 67, 1917-1927 (1986)]. However, even if recombinant Avipoxvirus having inserted therein antigenic gene is produced, as matter of fact, such Avipoxvirus would not be necessarily effective as vaccine. Under the situation, it is quite unknown if any vaccine for preventing NDV infection with the recombinant Avipoxvirus inserted with F gene of NDV could be produced. In recent years, F gene of human parainfluenza virus III was inserted into vaccinia virus; it is suggested that the cytotoxicity to recombinant virus-infected cells would be strengthened by expression of the inserted F gene [J. Virol., 61, 3416-3423 (1987)]. As stated above, F gene-introduced recombinant virus encounters problems that cell fusion with the virus is liable to occur, etc.

Therefore, the present inventors have made extensive investigations under the state of the art, aiming at construction of a recombinant Avipoxvirus capable of proliferation in which cDNA encoding NDV-derived antigen has been inserted into genome DNA. As a result, the present inventors have found that the recombinant Avipoxvirus capable of proliferation can be obtained by selecting the domain coding for F protein from cDNA of NDV, preferably cDNA of attenuated strain such as D26 strain and integrating such cDNA into the genome region non-essential to proliferation of Avipoxvirus. They have also found that these recombinant Avipoxviruses containing F gene are effective as vaccine that causes no fusion of infected cells but can prevent infections with both Avipoxvirus and NDV. The present invention has thus come to be accomplished.

Thus, according to the present invention, there is provided a recombinant Avipoxvirus in which NDV-derived antigen-encoding cDNA has been inserted into a genome region non-essential to proliferation of Avipoxvirus, preferably together with DNA having a promoter function, in the form capable of expression.

## BRIEF DESCRIPTION OF THE DRAWINGS

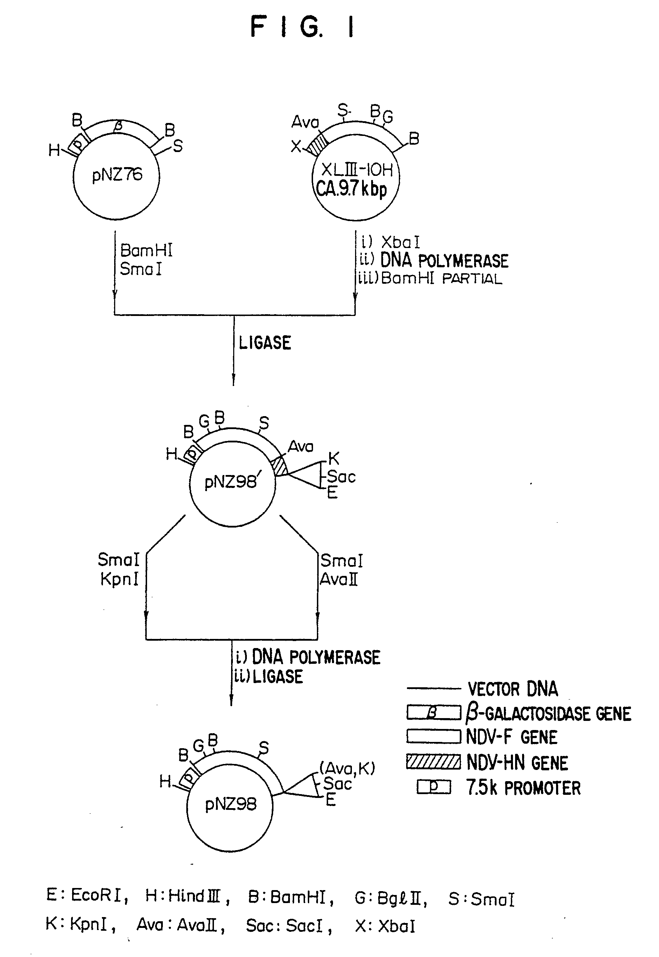

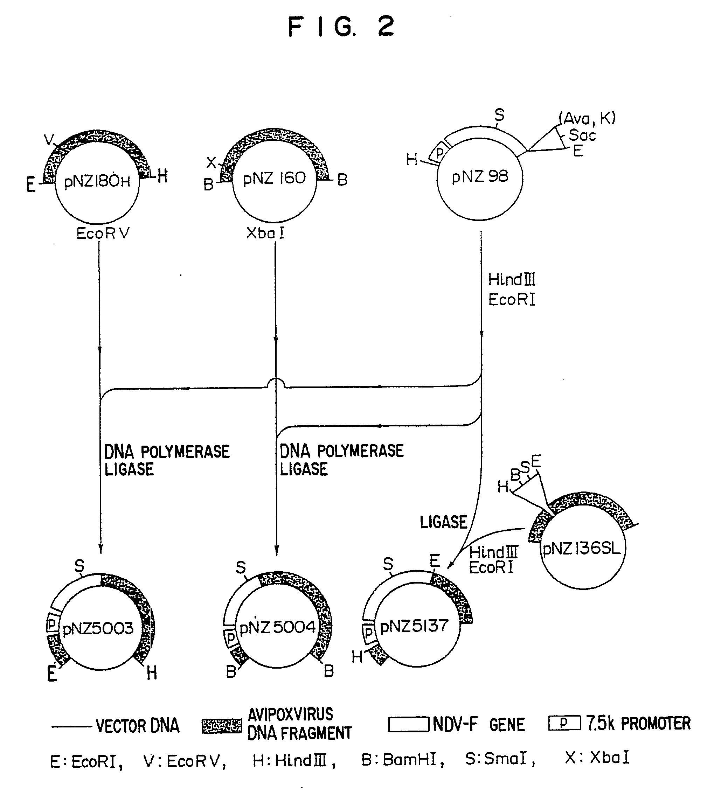

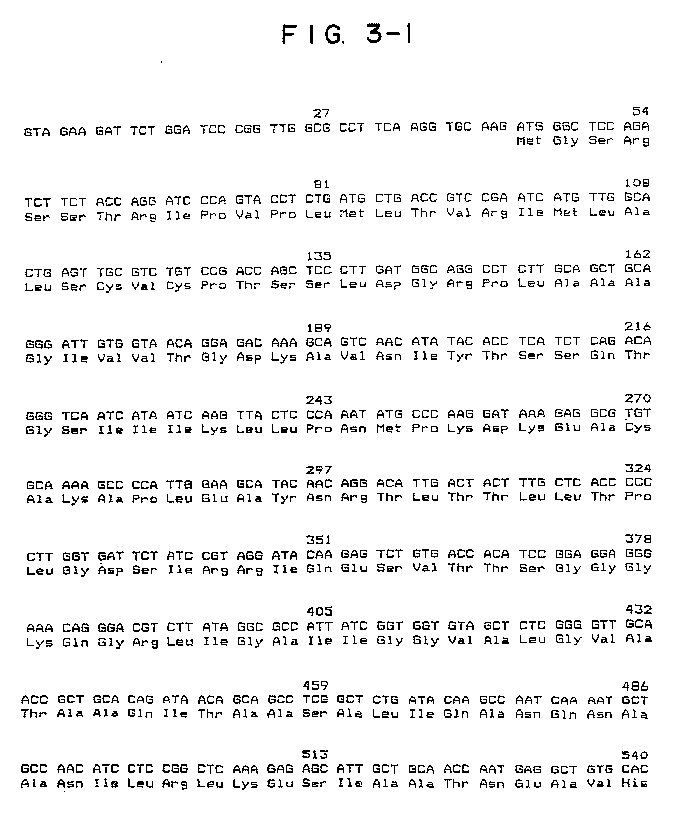

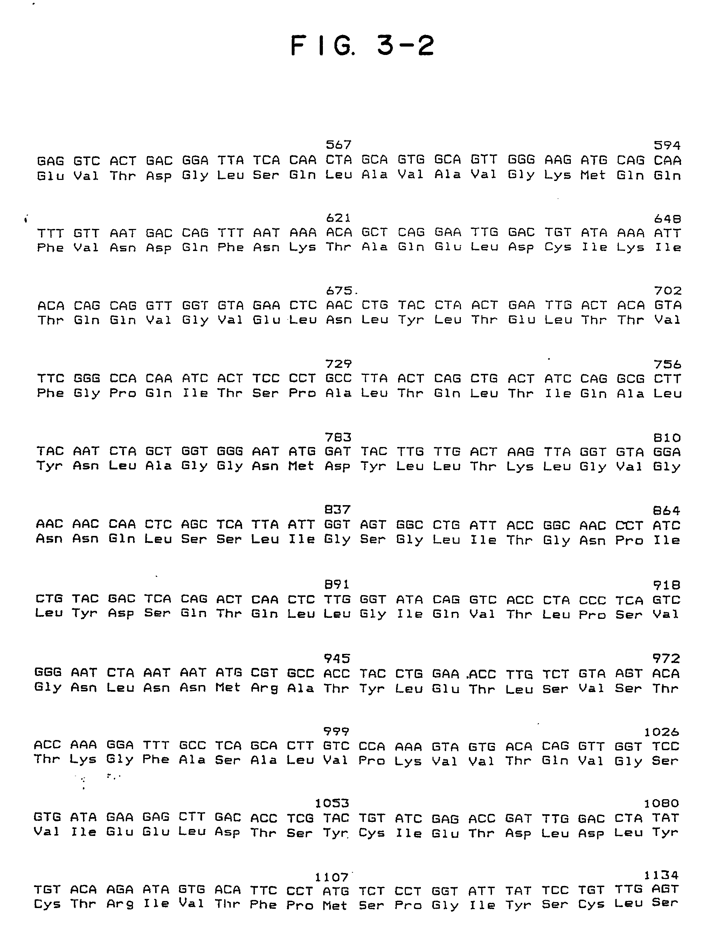

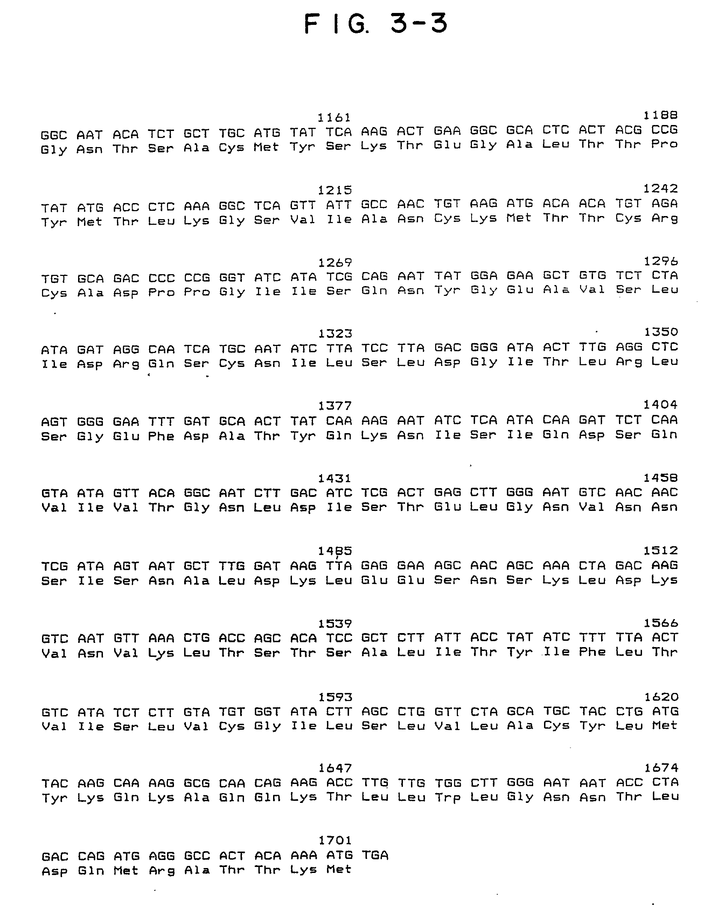

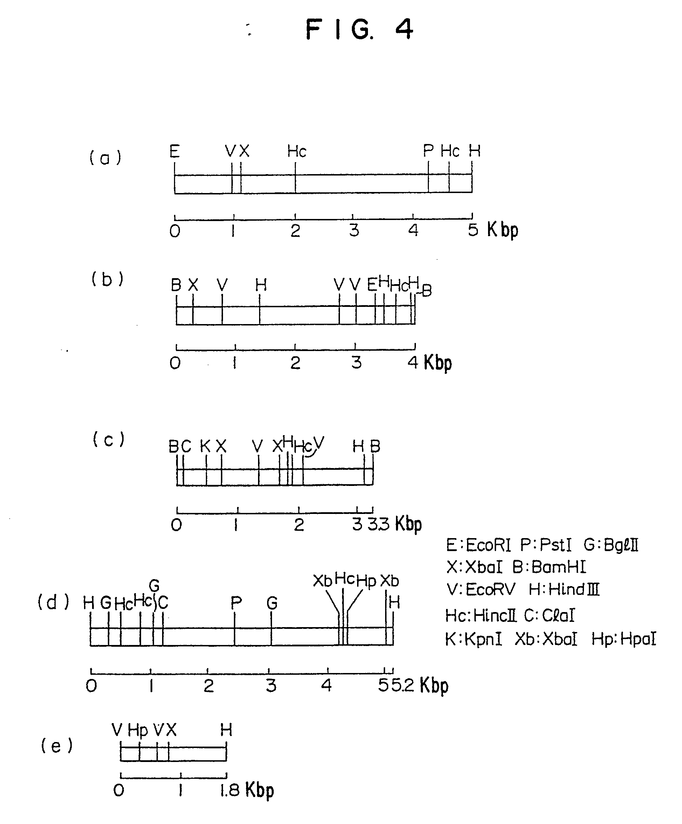

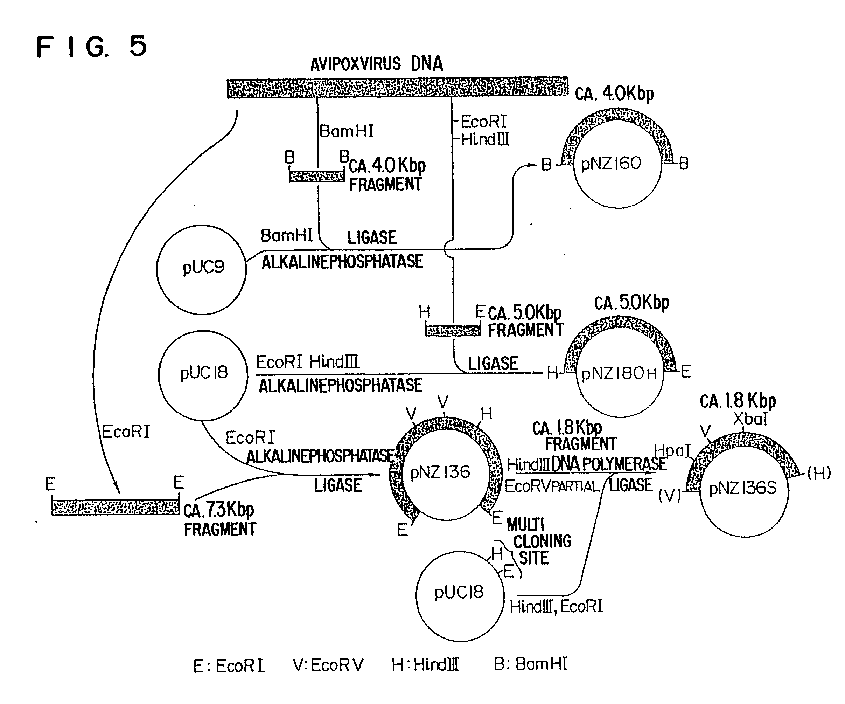

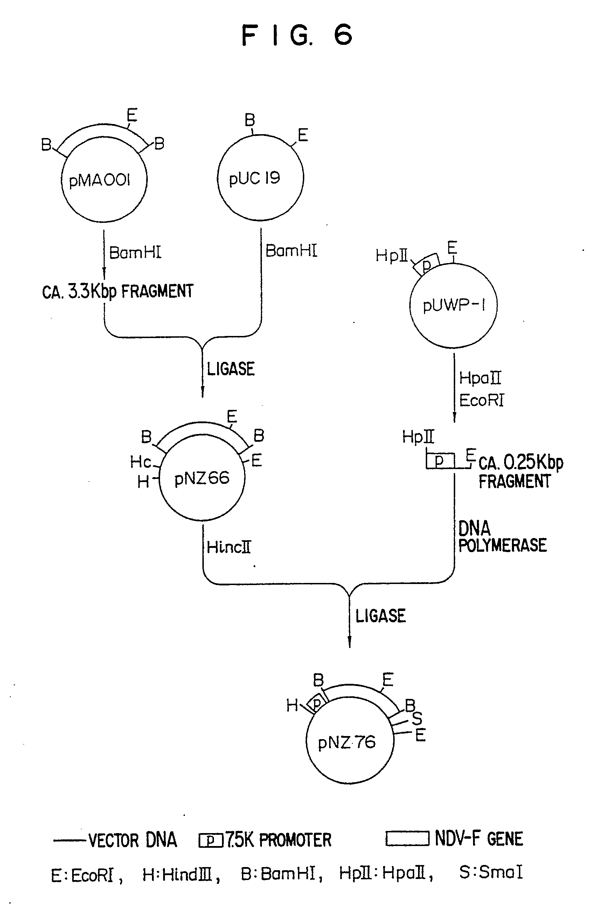

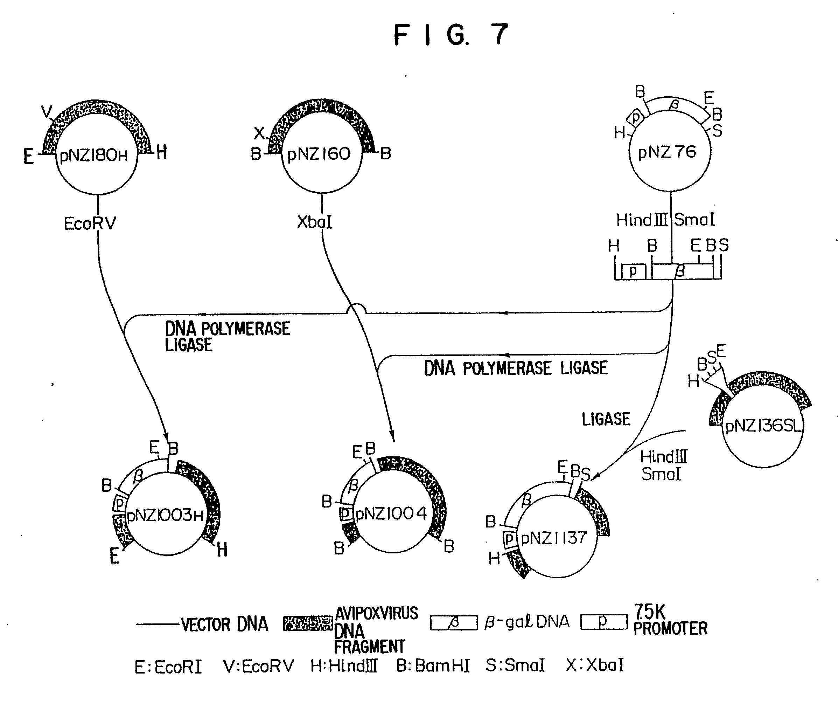

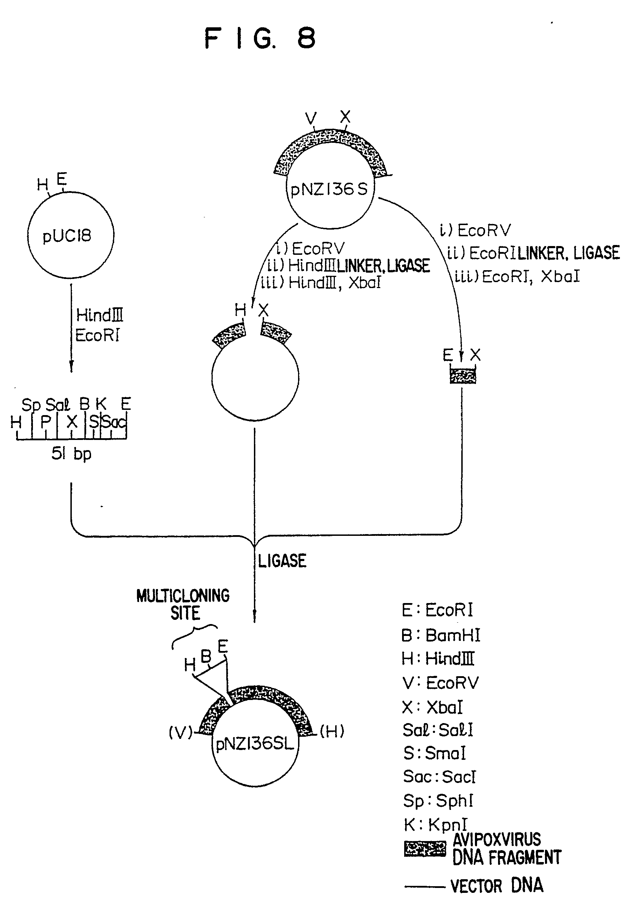

Figs. 1 and 2 show procedures for construction of recombinant Avipoxvirus of the present invention. Fig. 3 shows an amino acid sequence and nucleotide sequence of F gene from NDV. Fig. 4 shows an example of a DNA region non-essential to proliferation of Avipoxvirus. Fig. 5 shows procedures for construction of a plasmid vector containing at least a part of the DNA region non-essential to proliferation. Fig. 6 illustrates procedures for construction of hybrid plasmid (A) referred to in the present invention. Fig. 7 illustrates procedures for construction of hybrid plasmid (B) referred to in the present invention. Figure 8 illustrates procedures for construction of hybrid plasmid (C) referred to in the present invention.

## DETAILED DESCRIPTION OF PREFERRED EMBODIMENTS

Any virus is usable as the virus employed to insert NDV-derived cDNA in the present invention as far as it is classified into the genus Avipoxvirus but preferred are those capable of growing in cells of fowls such as chicken, turkey, duck, etc. Specific examples include Fowlpoxvirus such as ATCC VR-251, ATCC VR-250, ATCC VR-229, ATCC VR-249, ATCC VR-288, Nishigawara strain, Shisui strain, etc.; and those akin to Fowlpoxvirus and used to avian live vaccine strain such as NP strain (chick embryo habituated pigeonpoxvirus Nakano strain), etc. These strains are all commercially available and easily accessible.

Furthermore, NDV-derived F protein expresses in infected cells are expressed in the form of $F_0$ protein and in virulent strains such as Australia-Victoria strain (hereafter referred to as AV strain), etc., the strain is cleaved at about 1/5 from the N terminus to form two polypeptides of $F_1$ and $F_2$ and by their cleavage, fusion activity of infected cells occurs; whereas in attenuated strains such as D26 strain, etc., continuous basic amino acid residues immeditely before the site cleaved to $F_1$ and $F_2$ present in virulent strains are not present so that $F_0$ protein is not cleaved into $F_1$ and $F_2$. For this reason, there is little fusion activity in infected cells [Virus Research, 7, 241-255 (1987)]. In the present invention, cDNA coding for NDV-derived F protein can be prepared using attenuated strains, preferably D26 strain of NDV, etc., which cause no cleavage of $F_0$ protein into $F_1$ and $F_2$. Such cDNA may also be prepared using AV strain, etc. Where virulent strain-derived F gene such as AV strain is utilized, it is preferred to use the F gene after converting the portion immediately before the cleavage site of $F_0$ protein to $F_1$ and $F_2$ into attenuated type causing no cleavage, using synthetic DNA, etc. For example, cDNA prepared from D26 described above is composed of 1659 base pairs from 43 to 1701 in the nucleotide sequence shown in Fig. 3.

cDNA prepared from AV strain is also composed of 1659 base pairs and the sequence other than that around the cleavage site has high homology [Virus Research, 7, 241-255 (1987)].

As described above, the sequences of cDNA encoding NDV-derived F protein are not necessarily identical but in the present invention, cDNA may be modified (namely, its nucleotide sequence may be substituted, inserted or deleted), in such a fashion that cDNAs have substantially the same function as in the 2 kinds of cDNAs described above. Of course, insofar as they have substantially the same function, they may also be modified to such an extent that their amino acid sequences are different. However, it is preferred to use cDNA coding for polypeptide of attenuated type such as F protein of D26 strain which is not cleaved to $F_1$ and $F_2$. Of course, it is also possible to convert cDNA of virulent strain such as AV strain into attenuated type by replacing the portion around the cleavage sige with synthetic DNA, etc. and use the resulting cDNA of attenuated type.

Upon practice of the present invention, a first recombinant vector in which a DNA region non-essential to proliferation of Avipoxvirus has been inserted is firstly constructed. Preferred is a recombinant vector containing in the same region a promoter functioning in Avipoxvirus.

Specific examples of the DNA region non-essential to proliferation include regions which cause homologous recombination with EcoR I-Hind III fragment (about 5.0 kbp), BamH I fragment (about 4.0 Kbp), EcoR V-Hind III fragment (about 1.8 kbp), BamH I fragment (about 3.3 Kbp), Hind III fragment (about 5.2 Kbp), etc. (restriction enzyme map is as shown in Fig. 4).

Such a DNA region non-essential to proliferation can be fixed, for example, by the following procedures. First, hybrid plasmid (A) having inserted therein an optional DNA fragment from Avipoxvirus genome and hybrid plasmid (B) ligated with DNA encoding an enzyme capable of being readily detected under control of a promoter are prepared. Next, a DNA fragment fully containing DNA coding for the promoter and the enzyme is prepared from hybrid plasmid (B). By inserting the DNA fragment into a virus DNA portion of hybrid plasmid (A), hybrid plasmid (C) is prepared. Then, hybrid plasmid (C) is transfected into a host cell previously infected with Avipoxvirus, whereby positive or negative formation of recombinant Avipoxvirus is confirmed.

Selection to see if a recombinant Avipoxvirus is constructed by the series of procedures described above may be performed in a conventional manner. Where, for example, β-galactosidase gene is used as enzyme-coding DNA, after agarose medium containing chlorophenol red-β-D-galactopyranoside (hereafter referred to as CPRG) is overlaid, plaques are recognized on a medium for forming plaques of recombinant Avipoxvirus; by incubation at 37° C, plaques are stained red and the stained plaques may thus be selected.

As such, in case that the recombinant Avipoxvirus is obtained using detection of enzyme activity as a means for selection, it is indicated that the DNA fragment derived from vaccinia virus which is used to construct hybrid plasmid (A) is the DNA region non-essential to proliferation.

The DNA having a promoter function as used in the present invention may be any DNA having any nucleotide sequence as long as it can effectively function as a promoter in the transcription system possessed by Avipoxvirus, irrespective of synthesized or natural DNA. Needless to say, promoters intrinsic to Avipoxvirus such as a promoter of Avipoxvirus gene coding for thymidine kinase are available and, even DNA derived from virus other than Avipoxvirus or DNA derived from eucaryote or procaryote can be naturally used in the present invention, so long as it meets the requirement described above.

Concrete examples of these promoters include promoters of vaccinia virus illustrated in Journal of Virology, 51, 662-669 (1984), specifically, a promoter of vaccinia virus gene coding for 7.5 K polypeptide, a promoter of vaccinia virus gene coding for 19 K polypeptide, a promoter of vaccinia virus gene coding for 42 K polypeptide, a promoter of vaccinia virus gene coding for thymidine kinase polypeptide, a promoter of vaccinia virus gene coding for 28 K polypeptide, etc.

The promoter may be complete or partialy modified as long as it retains the function.

The first recombinant vector may be constructed in a conventional manner. For example, after inserting the DNA fragment non-essential to proliferation of Avipoxvirus into an appropriate vector, a promoter added with a restriction enzyme cleavage sequence may be incorporated at the downstream thereof, if necessary and desired, utilizing restriction enzyme cleavage sites present in the fragment.

Specific examples of the vector used herein include a plasmid such as pBR322, pBR325, pBR327, pBR328, pUC7, pUC8, pUC9, pUC19, etc.; a phage such as ! phage, M13 phage, etc.; a cosmid such as pHC79 (Gene, 11, 291, 1980) and the like.

In the present invention, the second recombinant vector is constructed by inserting the region encoding HN of NDV into the promoter of the first recombinant vector at the downstream thereof. Insertion may also be effected in a conventional manner. For example, NDV-derived cDNA fragment may be inserted, utilizing the restriction enzyme cleavage site previously provided at the downstream of the promoter of the first vector.

Upon construction of these first and second recombinant vectors, the method using Escherichia coli, by which genetic engineerings are easy may be used. The plasmid vector used is not particularly limited as far as it is suited for purposes.

In the present invention, next, the second recombinant vector is transfected into animal cultured cells previously infected with Avipoxvirus, to cause homologous recombination between the vector DNA and the virus genome DNA, whereby the recombinant Avipoxvirus is constructed. The animal cultured cells used herein may be any cells insofar as Avipoxvirus can grow there. Specific examples are chicken-derived culture cells such as chick embryo fibroblast (hereafter referred to as CEF), etc. Furthermore, chick chorio-allantoic membrane is also naturally included in the category of host cell.

Construction of the recombinant Avipoxvirus may be carried out in a conventional manner. The second recombinant vector treated by the calcium phosphate coprecipitation method is transfected into host cells infected with Avipoxvirus according to, for example, D.M. Glover, DNA Cloning, volume II, A Practical Approach, pp. 191-211, IRL Press, Oxford, Washington. The thus obtained virus mass containing recombinant virus is infected to host cells cultured on Eagle's MEM. Plaques grown on the medium may be made recombinant virus candidate strains. For selection of a virus having inserted therein the DNA fragment coding for F of NDV from these candidate strains, the candidate strains may be purified by a hybridization method using the DNA encoding F as a probe and immunoassay using an anti-NDV antibody is then applied.

Therefore, according to the present invention, there is provided the recombinant Avipoxvirus which is utilizable as a live vaccine for fowls and in which NDV-derived antigen-coding cDNA has been inserted into the genome region non-essential to proliferation of Avipoxvirus, together with the DNA having promoter functions, in the form capable of expression.

(Examples)

The present invention will be described in more detail with reference to the examples below.

Reference Example 1 (Identification of non-essential DNA region)

(1) Preparation of Avipoxvirus genome DNA:

Avipoxvirus NP strain (chick embryo habituated pigeonpoxvirus Nakano strain, manufactured by Japan Pharmacy Co., Ltd.) was inoculated on chick embryo fibloblasts cultured in a 75 cm² culture flask in 1 p.f.u./cell. After culturing in an incubator at 37°C in 5% CO₂ for 2 hours, 15 ml of Eagle's MEM supplemented with 10% Tryptose phosphate broth (Difco Co., Ltd.) and 0.03% L-glutamine was added followed by incubation in an incubator for 4 days at 37°C in 5% CO₂. Then the culture supernatant was centrifuged at 3000 r.p.m. for 10 minutes to recover the supernatant. The supernatant was centrifuged at 25000 r.p.m. (about 98000 x g) for an hour and the precipitates were recovered. The precipitates were suspended in a DNase in a reaction buffer (50 mM Tris, pH 7.5, 1 mM MgCl₂) of a 1/10 volume of the culture supernatant and DNase I (Boehringer Mannheim Co., Ltd.) was added to the suspension in 9 μg/ml followed by reacting at 37°C for 30 minutes. After the reaction, 25 mM EDTA.2Na was added and the mixture was allowed to stand at room temperature for 30 minutes. Thereafter, 500 μg/ml of proteinase K (Boehringer Mannheim Co., Ltd.) and 1% final concentration of sodium dodecyl sulfate (SDS) were added followed by reacting at 37°C overnight. After gently treating with phenol-chloroform, the reaction mixture was precipitated with ethanol to give 0.5 μg of virus DNA.

(2) Construction of hybrid plasmid (A) containing Avipoxvirus genome fragment (cf. Figure 5)

(a) Construction of plasmid (pNZ180H) containing about 5.0 Kbp EcoR I-Hind III fragment of DNA from Avipoxvirus NP strain:

After digesting 2 μg of pUC18 (manufactured by Pharmacia Inc.) with EcoR I and Hind III, extraction was performed with phenol-chloroform (1 : 1) and recovered cleaved pUC18 by precipitation with ethanol. The 5'-end phosphate was removed by treating with alkali phosphatase. After extraction again with phenol-chloroform, DNA was recovered by precipitation with ethanol. The cleaved pUC18, 0.2 μg, and the digestion product from 1 μg of the purified Avipoxvirus (NP strain) DNA with EcoR I and Hind III were ligated with each other. Competent E. coli JM 103 was transformed and cultured at 37°C for 15 hours in LB agar medium supplemented with 0.003% of 5-bromo-4- chloro-3-indolyl-β-D-galactopyranoside, 0.03 mM of isopropyl-β-D-galactopyranoside and 40 μg/ml of ampicillin. After the transformed E. coli grown on agar medium, white colony was cultured at 37°C for 15 hours in LB liquid medium supplemented with 40 μg/ml of ampicillin and plasmid was extracted by the method of Birnboim and Doly [Nucleic Acid Research, 7, 1513 (1979)]. After digesting with EcoR I and Hind III, a hybrid plasmid bearing the fragment having the same length as that of the original Avipoxvirus DNA EcoR I-Hind III fragment was detected by 0.6% agarose electrophoresis, which was named pNZ180H. A restriction enzyme map of the about 5.0 Kbp EcoR I-Hind III fragment is shown in Fig. 4 (a). pNZ180H corresponds to hybrid plasmid (A).

(b) Construction of plasmid (pNZ160) containing about 4.0 Kbp BamH I fragment of DNA from Avipoxvirus NP strain:

A hybrid plasmid was obtained in a manner similar to (a) except that vector pUC18 used in (a) was changed to pUC9 and about 4.0 Kbp BamH I fragment of DNA from NP strain was used in lieu of the EcoR I-Hind III fragment. This hybrid plasmid was named pNZ160. A restriction enzyme map of about 4.0 Kbp BamH I fragment is shown in Fig. 4 (b). pNZ160 corresponds to hybrid plasmid (A).

(c) Construction of plasmid (pNZ136S) containing about 1.8 Kbp EcoR V-Hind III fragment of DNA from Avipoxvirus NP strain:

A hybrid plasmid was obtained in a manner similar to (a) except that about 7.3 Kbp EcoR I fragment of DNA from NP strain was used in lieu of about 5.0 Kbp EcoR I-Hind III fragment used in (a). This hybrid plasmid was named pNZ136. Furthermore, after pNZ136 was digested with Hind III, the digestion product was partially digested with EcoR V. The partial digestion product was subjected to 0.8% agarose electrophoresis and recover about 1.8 Kbp EcoR V-Hind III fragment composed of about 0.6 Kbp EcoR V fragment and about 1.2 Kbp EcoR V-Hind III fragment adjacent to each other. On the other hand, after pUC18 was digested with Hind III and EcoR I, extraction was performed with phenol : chloroform (1 : 1) and the extract was precipitated with ethanol. The cleaved pUC18 was recovered by agarose gel electrophoresis. The about 1.8 Kbp EcoR V-Hind III fragment was rendered blunt end with DNA polymerase followed by ligation with ligase. Competent E. coli TG1 was transformed and the transformants were selected on ampicillin-supplemented LB agar medium to give a plasmid containing about 1.8 Kbp EcoR V-Hind III fragment. The plasmid was named pNZ136S.

A restriction enzyme map of about 1.8 Kbp EcoR V-Hind III fragment is shown in Fig. 4 (e) and pNZ136S corresponds to hybrid plasmid (A).

(3) Construction of hybrid plasmid (B) (pNZ76) ligated with a promoter and DNA coding for readily detectable enzyme under its control (cf. Fig. 6)

A plasmid having inserted about 0.26 Kbp of Sal I-Rsa I fragment containing a promoter of DNA coding for 7.5 K dalton peptide of vaccinia virus WR strain [Cell, 125, 805-813 (1981)] into the Sal I-Sma I portion of pUC9 was named pUWP-1.

After digesting 10 μg of pMA001 [Shirakawa et al., Gene, 28, 127 (1984)] with BamH I, β-galactosidase gene (about 3.3 Kbp) was recovered in a manner similar to (2). On the other hand, after digesting 0.3 μg of pUC19 with BamH I, extraction was performed with phenol-chloroform and recovery was made through precipitation with ethanol. By ligation with the β-galactosidase gene prepared as described above, hybrid plasmid pNZ66 was constructed.

On the other hand, 40 μg of pUWP-1 was digested with Hpa II and EcoR I and, a fragment of about 0.26 Kbp containing 7.5 K promoter was separated by 1.5% low melting point agarose electrophoresis (70 volts, 6 hours) and DNA was recovered by operation similar to (2). The cohesive end of this DNA fragment was made the blunt end by DNA polymerase. After 0.3 μg of pNZ66 was digested with Hinc II, extraction was performed with phenol-chloroform and recovery was made through precipitation with ethanol. By ligation with about 0.26 Kbp of the 7.5 K promoter gene described above, a hybrid plasmid was obtained and it was named pNZ76. pNZ76 corresponds to hybrid plasmid (B).

(4) Construction of hybrid plasmid (C) from hybrid plasmid (A) and hybrid plasmid (B) (cf. Fig. 7)

(a) Construction of hybrid plasmid (pNZ1003H) having inserted the ligation fragment of vaccinia virus 7.5 K promoter and β-galactosidase DNA into EcoR V site of pNZ180H

After digesting 10 μg of pNZ76 with Hind III and Sma I, a fragment of about 3.6 Kbp was separated by 0.7% low melting point agarose electrophoresis (40 volts, 20 hours). After the DNA fragment was confirmed by staining with ethidium bromide, gel was cut out, treated with phenol and precipitated with ethanol to recover the DNA fragment.

On the other hand, 1 μg of pNZ180H was digested with EcoR V, extracted with phenol-chloroform and precipitated with ethanol to recover the same. The cleaved pNZ180H DNA, 0.3 μg, was mixed with about 0.4 μg of the about 3.6 Kbp fragment (ligation fragment of 7.5 K promoter DNA and β-galactosidase gene) and, the cohesive end was made the blunt end by DNA polymerase. After extracting with phenol-chloroform, the DNA was recovered. The recovered DNA was ligated with ligase. Competent E. coli TG1 strain was transformed and allowed to grow at 37°C for 15 hours in LB agar medium supplemented with 40 μg/ml of ampicillin. A plasmid was recovered from E. coli grown in a manner similar to (2) and digested with BamH I. A hybrid plasmid containing the β-galactosidase gene fragment (about 3.3 Kbp) was selected by 0.5% agarose electrophoresis, which was named pNZ1003H.

(b) Construction of hybrid plasmid (pNZ1004) having inserted the ligation fragment of vaccinia virus 7.5 K promoter and β-galactosidase DNA into the Xba I site of pNZ160

A hybrid plasmid containing the β-galactosidase gene fragment (about 3.3 Kbp) was selected in a manner similar to (a) except that restriction enzyme EcoR V used in (a) was replaced with Xba I. This hybrid plasmid was named pNZ1004.

6

(c) Construction of hybrid plasmid (pNZ1137) having inserted the ligation fragment of vaccinia virus 7.5 K promoter and $\beta$-galactosidase DNA into the EcoR V site of pNZ136S

After digesting pNZ136S with EcoR V, EcoR I linker (manufactured by Takara Shuzo Co., Ltd.) was ligated to the digestion product with ligase. By extraction with phenol-chloroform and precipitation with ethanol, DNA was recovered. After the recovered DNA was likewise digested with EcoR I and Xba I, the digestion product was subjected to 2.0% agarose gel electrophoresis to recover about 140 bp EcoR I-Xba I fragment. Also in a similar manner, after digesting pNZ136S with EcoR V, Hind III linker (manufactured by Takara Shuzo Co., Ltd.) was ligated to the digestion product with ligase. By extraction with phenol-chloroform and precipitation with ethanol, DNA was recovered. After the recovered DNA was digested with Hind III and Xba I, the digestion product was subjected to 0.8% agarose gel electrophoresis to recover about 4.3 Kb Hind III-Xba I fragment. Furthermore, after digesting pUC18 with Hind III and EcoR I, 51 bp fragment of polylinker portion was recovered from 2.0% agarose gel. The about 140 bp EcoR I-Xba I fragment, about 4.3 Kbp Hind III-Xba I fragment and 51 bp Hind III-EcoR I fragment thus obtained were ligated with each other by ligase. A plasmid was extracted in a manner similar to Example 1 (4). The plasmid in which 3 fragments had been ligated was detected and this was named pNZ136SL (cf. Fig. 8).

On the other hand, after digesting pNZ76 with Hind III and Sma I, about 3.6 Kbp fragment was recovered in a manner similar to (2). The pNZ136SL described above was digested with Hind III and Sma I. By extraction with phenol-chloroform and precipitation with ethanol, recovery was performed. Both were ligated with each other by ligase and competent E. coli TG1 strain was transformed. Subsequently in a manner similar to (2), a hybrid plasmid was selected and this was named pNZ1137.

(5) Construction of recombinant Avipoxvirus:

Avipoxvirus NP strain was inoculated on chick embryo fibloblast cultured in a 25 cm² culture flask in 0.05 p.f.u./cell. The hybrid plasmid, 50 $\mu$g, obtained in (4) was dissolved in 2.2 ml of sterile water and, 2.5 ml of a mixture of 1% HEPES and 0.6% sodium chloride and 50 $\mu$l of buffer solution obtained by mixing 70 mM sodium dihydrogenphosphate 12 hydrate and 70 mM sodium dihydrogenphosphate 2 hydrate in equal volumes were mixed with the solution to prepare an aqueous solution. The aqueous solution was transferred to a 15 ml tube (manufactured by Falcon Co., Ltd.) and 300 $\mu$l of 2 M calcium chloride aqueous solution was dropwise added thereto while agitating with a stirrer to form DNA-calcium phosphate coprecipitates. Forty five minutes after the inoculation of virus, 0.5 ml of the coprecipitates were dropwise added to the infected CEF. After settling in an incubator at 37° C in 5% $CO_2$ for 30 minutes, 4.5 ml of Eagle's MEM supplemented with 5% fetal bovine serum, 0.03% L-glutamine and 10% Tryptose phosphate broth was added thereto. Three hours after, the culture supernatant was exchanged and cultured in an incubator for 3 days at 37° C in 5% $CO_2$. The system including culture cells was frozen and thawed 3 times to give a solution of virus containing the recombinant.

(6) Selection of the recombinants by CPRG

The virus solution obtained in (5) was inoculated on CEF cultured in a 10 cm Petri dish. Two hours after, 10 ml of phenol red-free Eagle's MEM supplemented with 0.8% Bacto agar (manufactured by Difco Co., Ltd.), 5% fetal bovine serum, 0.03% L-glutamine and 10% Tryptose phosphate broth was put thereon in layers followed by incubation in an incubator at 37° C in 5% $CO_2$ for 3 days. On the medium was put 10 ml of phenol red-free Eagle's MEM supplemented with 0.8% Bacto agar, 0.03% L-glutamine, 10% Tryptose phosphate broth and 0.03% CPRG (manufactured by Boehringer Mannheim) in layers followed by incubation in an incubator at 37° C in 5% $CO_2$ for 6 hours. The recombinant virus expresses $\beta$-galactosidase and changes CRPG to red so that both agar and cells around the recombinant plaque became red and could be easily distinguished over non-recombinant. The recombinant formed from this red plaque was isolated with a sterilized Pasteur pipette. Each recombinant was named as shown in Table 1.

Table 1

| Parent Strain | Hybrid Plasmid (C) | Recombinant |
|---|---|---|
| Avipoxvirus NP strain | pNZ1003H | fNZ1003H |
| Avipoxvirus NP strain | pNZ1004 | fNZ1004 |
| Avipoxvirus NP strain | pNZ1137 | fNZ1137 |

(7) Analysis of genome DNA of the recombinant Avipoxvirus:

After each Avipoxvirus obtained in (6) was subjected to plaque purification, the Avipoxvirus was inoculated on CEF cultured in a 10 cm Petri dish in 1 p.f.u./cell. Subsequently in a manner similar to (1), virus DNA was isolated. After 2 μg of each recombinant virus DNA was digested with BamH I or Hind III, fragments were separated by 0.5% agarose electrophoresis (25V, 20 hours). After the DNA was transferred to a nylon membrane by the Southern's method [Journal of Molecular Biology, 98. 503 (1979)], the DNA was immobilized onto the nylon membrane at 80°C under reduced pressure. After treating with 4-fold SET-[0.6 M NaCl, 0.08 M Tris HCl (pH 7.8), 4 mM EDTA]-10-fold Denhardt-0.1% SDS at 68°C for 2 hours, β-galactosidase DNA labelled with $^{32}$P was hybridized with 50 μg/ml of 4-fold SET-10-fold Denhardt-0.1% SDS-0.1% Na$_4$P$_2$O$_7$-modified salmon sperm DNA at 68°C for 14 hours by nick translation. After washing and drying, the nylon membrane was put on an X ray film to perform autoradiography, whereby the presence of bands was confirmed. It was confirmed that these recombinant viruses contained the β-galactosidase genes in definite locations.

(8) Identification of non-essential DNA region

From the foregoing results, it will be understood that the EcoR I-Hind III fragment (about 5.0 Kbp) used for construction of pNZ180H, BamH I fragment (about 4.0 Kbp) used for construction of pNZ160 and EcoR V-Hind III fragment (about 1.8 Kbp) used to construct pNZ136S are regions non-essential to proliferation of virus.

In a manner similar to the above, it has also been confirmed that BamH I fragment (about 3.3 Kbp) and Hind III fragment (about 5.2 Kbp) of DNA from the NP strain are also regions non-essential to proliferation of virus. A restriction enzyme map of these fragments is as shown in Fig. 4.

Furthermore, hybrid plasmids pNZ1003H, pNZ1004 and pNZ1137 constructed in (4) all contain the DNA region non-essential to growth of Avipoxvirus and can thus be utilized as the first recombinant vector in the present invention.

Example 1 Construction of the second recombinant vector (pNZ5003) containing F gene DNA of NDV (cf. Figs. 1 and 2)

(1) Construction of hybrid plasmid (pNZ98) ligated with a promoter and F gene DNA under its control (cf. Fig. 1):

Plasmid XLIII-10H containing cDNA of F and HN genes from NDV [Virus Research, 7, 241-255 (1987)] was obtained from Assistant Professor Masao Kawakita, of Tokyo University, Department of Liberal Arts. The nucleotide sequence of this plasmid was analyzed by the dideoxy method using M13 phage and, it has been revealed by contrasting to the sequence of known F gene [Virus Research, 5, 343-356 (1986)] that cDNA of F gene is 1659 base pairs from 43 to 1701 shown in Fig. 3. This clarification was made by Assistant Professor Kawakita.

The amino acids for which the cDNA obtained herein codes had 95% or more homology to the amino acid sequence of F protein from AV strain. It is revealed that in AV strain, however, the cleavage site from F$_0$ to F$_1$ and F$_2$ is between Arg-Phe in -Arg-Arg-Gln-Lys-Arg-Phe-Ile, whereas in D26 strain, the cleavage site is between Arg-Leu in -Gly-Lys-Gln-Gly-Arg-Leu-Ile- (provided that D26 strain is not cleaved into $F_1$ and $F_2$ from $F_0$) and any continuous basic amino acid residues just before the cleavage site is absent.

After digesting 4 μg of plasmid XLIII-10H with Xba I, the cohesive end was changed to the blunt end with DNA polymerase. After extracting with phenol-chloroform and then precipitation with ethanol, DNA was recovered. After partial digesting the recovered DNA with BamH I, the digestion product was subjected to 0.8% agarose gel electrophoresis. About 2.1 Kbp fragment fully containing F gene was recovered. On the other hand, hybrid plasmid (B) (pNZ76) prepared in Reference Example 1 (3), with which promoter and DNA encoding readily detectable enzyme under its control had been ligated, was digested with BamH I and Sma I and, about 3.0 Kbp BamH I-Sma I fragment except for the lacZ gene portion was recovered. The about 3.0 Kbp fragment was ligated with the about 2.1 Kbp fragment described above by ligase and, competent E. coli TG1 strain was transformed. From the colony grown on LB agar medium supplemented with 50 μg/ml of ampicillin, plasmids were prepared and the desired clone was confirmed by cleavage with restriction enzymes. The plasmid was named pNZ98'. The plasmid pNZ98' contains about 300 bp of 5' end of HN gene, in addition to the full length of F gene. In order to remove the 5' end, pNZ98' was digested with Sma I and Kpn I and, about 4150 bp Sma I-Kpn I fragment was recovered by 0.8% agarose gel electrophoresis. Likewise, pNZ98' was digested with Sma I and Ava II and, about 650 bp Sma I-Ava II fragment was recovered by 1.5% agarose gel electrophoresis. These two fragments were mixed and the cohesive end was changed to the blunt end by DNA polymerase. Thereafter, the fragments were ligated with each other by ligase and, competent E. coli TG1 strain was transformed. From the colony grown on LB agar medium supplemented with 50 μg/ml of ampicillin, plasmids were prepared. The plasmid which could be again cleaved with restriction enzyme Sma I was selected. This plasmid was named pNZ98.

(2) Construction of the second recombinant vector (pNZ5003) from hybrid plasmid pNZ180H containing Avipoxvirus genone fragment and hybrid plasmid pNZ98 (cf. Fig. 2):

The second recombinant vector (pNZ5003), in which the ligated fragment of 7.5 K promoter of vaccinia virus and F gene had been inserted, was prepared in a manner similar to the production of hybrid plasmid pNZ1003H in Reference Example 1 (4) (a), except that plasmid pNZ98 was used instead of pNZ76 used in Reference Example 1 (4) and the digestion was performed with Hind III and EcoR I, instead of Hind III and Sma I.

Example 2 Construction of the second recombinant vector (pNZ5004) containing F gene DNA of NDV (cf., Fig. 2):

The second recombinant vector (pNZ5004), in which the ligated fragment of 7.5 K promoter of vaccinia virus and F gene had been inserted, was prepared in a manner similar to the production of hybrid plasmid pNZ1004 in Reference Example 1 (4) (b), except that plasmid pNZ98 was used instead of pNZ76 used in Reference Example 1 (4) and the digestion was performed with Hind III and EcoR I, instead of Hind III and Sma I.

Example 3 Construction of the second recombinant vector (pNZ5137) containing F gene DNA of NDV (cf., Fig. 2):

After digesting plasmid pNZ98 with Hind III and EcoR I, about 2.1 Kbp Hind III-EcoR I fragment ligated with 7.5 K promoter of vaccinia virus and F gene was recovered by 0.8% agarose gel electrophoresis. On the other hand, plasmid pNZ136SL used in Reference Example 1 (4) (c) was similary digested with Hind III and EcoR I. The digestion product was recovered by extraction with phenol-chloroform and precipitation with ethanol. The both fragments were ligated with each other by ligase and, competent E. coli TG1 strain was transformed. From the resulting ampicillin-resistant strains, plasmids were prepared and, hybrid plasmid pNZ5137, of which the ligation fragment of 7.5 K promoter of vaccinia virus and F gene had been inserted into a part of the Avipoxvirus genome fragment, was selected.

Example 4 Construction of recombinant Avipoxvirus containing F gene of NDV:

A solution of the recombinant Avipoxvirus containing F gene of NDV was obtained in a manner similar to Reference Example 1 (5) except that the second recombinant vectors obtained in Examples 1, 2 and 3 were used instead of the hybrid plasmid used in Reference Example 1 (5).

Example 5 Selection of recombinant by plaque hybridization

The virus solution obtained in Example 4 was inoculated on CEF cultured in a 10 cm Petri dish. Two hours after, 10 ml of Eagle's MEM supplemented with 0.8% Bacto agar, 5% fetal bovine serum, 0.03% L-glutamine and 10% Tryptose phosphate broth was overlaid in layers followed by incubation in an incubator at 37°C in 5% $CO_2$ for 3 days. On the medium was further overlaid in layers ml of Eagle's MEM having the same composition as described above followed by incubation in an incubator at 37°C in 5% $CO_2$ for 3 days. Furthermore, 10 ml of Eagle's MEM supplemented with 0.8% Bacto agar, 0.03% L-glutamine, 10% Tryptose phosphate broth and 0.01% of neutral red was overlaid in layers followed by incubation in an incubator at 37°C in 5% $CO_2$ for 12 hours to stain the infected cells.

The agar medium was withdrawn from the Petri dish. A sterile nylon membrane was pushed onto the surface of the cells kept at 4°C and remained at the bottom of the Petri dish to transfer the viruses thereon. After repeating the treatment with 0.5 N NaOH for 10 minutes and with 1 M Tris-hydrochloride buffer for 5 minutes three times, the membrane was treated with 1.5 M NaCl and 0.5 M Tris-hydrochloride buffer for 5 minutes. The membrane was saturated with 2-fold SSC [1-fold SSC is 0.15 M NaCl and 0.015 M $C_3H_4(OH)(COONa)_3$] and sintered at 80°C for 2 hours. The membrane was treated with 4-fold SET (0.6 M NaCl, 0.08 M Tris HCl, 4 mM EDTA, pH 7.8)-10-fold Denhardt-0.1% SDS at 68°C for 2 hours. 4-Fold SET-10-fold Denhardt-0.1% SDS-0.1% $Na_4P_2O_7$-50 μg/ml-modified salmon sperm DNA and cDNA coding for F protein from NDV labeled with $^{32}P$ were hybridized by nick translation at 68°C for 14 hours. After washing, the nylon membrane was put on an X ray film, which was subjected to autoradiography to confirm the presence of a spot. The X ray film was overlaid on the agar kept at 4°C and a plaque coincided with the spot was identified to be a recombinant containing F gene. The recombinant was isolated with a sterilized Pasteur pipette. Three plaques of this recombinant appeared per one Petri dishes of 10 cm in which approximately 600 plaques appeared (about 0.5%).

With respect to the thus obtained recombinant viruses, the recombinant virus obtained using pNZ5003 was named fNZ5003. Likewise, those obtained using pNZ5004 and pNZ5137 were named fNZ5004 and fNZ5137, respectively.

Example 6 Purification of recombinant Avipoxvirus containing F gene of NDV

The plaque isolated in Example 5 was suspended in 1 ml of Eagle's MEM and 200 μl of the suspension was inoculated on CEF cultured in a 10 cm Petri dish. Two hours after, 10 ml of Eagle's MEM supplemented with 0.8% Bacto agar, 5% fetal bovine serum, 0.03% L-glutamine and 10% Tryptose phosphate broth was overlaid thereon in layers followed by incubation in an incubator at 37°C in 5% $CO_2$ for 3 days. On the medium was overlaid in layers 10ml of Eagle's MEM having the same composition described above followed by incubation in an incubator at 37°C in 5% $CO_2$ for 3 days. Ten ml of Eagle's MEM supplemented with 0.8% Bacto agar, 0.03% L-glutamine and 10% Tryptose phosphate broth was overlaid in layers on the medium followed by incubation in an incubator at 37°C in 5% $CO_2$ for 6 hours. The recombinant plaque formed a spot on an X ray film as in Example 5.

The foregoing operations were repeated in a similar manner to purify the recombinant again.

As the result, about 20 (about 0.3%) recombinant plaques appeared with fNZ5003, per 10 Petri dishes of 10 cm, in which approximately 600 plaques per dish appeared in Example 5; in the first plaque purification, about 100 (17%) of the recombinant plaques appeared in 3 Petri dishes in which about 200 plaques per dish appeared. In the second plaque purification, almost all plaques were the recombinants. Also with respect to fNZ5004 and fNZ5137, almost the same results were obtained.

Example 7 Expression of the recombinant in cells (immunofluorescent antibody technique)

The virus strains (fNZ5003, fNZ5004 and fNZ5137) of the present invention showing m.o. i of 1.0 or 0.1 were inoculated on CEF cultured in a chamber slide for tissue culture. After culturing at 37°C in a 5% $CO_2$ incubator for 2 hours, Eagle's MEM supplemented with 10% Tryptose phosphate broth (Difco Co., Ltd.) and 0.03% L-glutamine was added. Thereafter, incubation was performed for 2 days at 37°C in a 5% $CO_2$ incubator and the medium was then withdrawn. After washing with PBS (phosphate buffered saline) and air drying, the system was treated with cold acetone for 20 minutes to immobilise the cells. By examining in accordance with immunofluorescent technique using as a primary antibody anti-NDV chick antibody and as a secondary antibody fluorescein isothiocyanate-bound anti-chicken IgG antibody, it was confirmed that the recombinant Avipoxviruses had F protein productivity of NDV.

Example 8 Expression of recombinant in cells (enzyme antibody technique)

The virus solution obtained in Example 6 was inoculated on CEF cultured in a 10 cm Petri dish. Two hours after, 10 ml of Eagle's MEM supplemented with 0.8% Bacto agar, 5% fetal bovine serum, 0.03% L-glutamine and 10% Tryptose phosphate broth was overlaid in layers followed by incubation in an incubator at 37°C in 5% $CO_2$ for 3 days. Furthermore, 10 ml of Eagle's MEM having the same composition as described above was overlaid in layers followed by incubation in an incubator at 37°C in 5% $CO_2$ for 3 days.

The agar medium was withdrawn from the Petri dish. A sterilized nylon membrane was pushed onto the surface of cells remained at the bottom of the Petri dish to transfer virus plaques and cells thereon. After treating with PBS (phosphate buffered saline) containing 2% skimmed milk for 30 minutes, anti-NDV chicken antibody was reacted as a primary antibody at room temperature for an hour. After the reaction, the mixture was washed with PBS and biotinated anti-chicken IgG antibody was reacted as a secondary antibody at room temperature for an hour. After the reaction, the mixture was washed with PBS and horse radish peroxidase-bound avidin-biotin complex was reacted at room temperature for 30 minutes. After the reaction, a color was formed with 8 mM Tris-hydrochloride buffer (pH 8.0) containing 0.05% 4-chloro-1-naphthol and 0.3% $H_2O_2$. By checking with the foregoing enzyme antibody method, it was confirmed that the recombinant Avipoxviruses had F protein NDV productivity.

Example 9 Immune effect of the recombinant Avipoxvirus and effect of preventing infection

The recombinant Avipoxvirus fNZ5137 was inoculated on CEF cultured in 5 flasks having 150 $cm^2$ each followed by culturing at 37°C for 48 hours. After freezing and thawing CEF twice, cells were collected and centrifuged at 2,000 rpm for 15 minutes and the supernatant was collected. After adding 40 ml of 20% sodium glutamate solution and 30 ml of 10% saccharose solution to 130 ml of the supernatant, 2 ml each of the mixture was separately charged. Freeze-drying was carried out for 30 hours under reduced pressure, after preliminary freezing for 1.5 hours. One vial of dry vaccine was dissolved in 10 ml of 30 % glycerine-supplemented physiological saline and the solution was inoculated to the right wing webb of SPF chicken of 7 days age in a dose of 0.01 ml per one chick, using a pierce needle. After the inoculation, the state of pox formation was observed. After hemagglutination inhibiting (hereafter referred to as HI) antibody to Newcastle disease (NDV) was determined, virulent chicken pox virus Nishigawara strain and virulent NDV Sato strain were challenged to determine the immune effect of the vaccine.

The HI reaction of Newcastle disease virus was determined by equal volume of NDV antigen containing 4 hemagglutination units to 25 μl of 2-fold serial dilution of serum to be tested, mixing them, settling the mixture for 10 minutes for sensitization, then adding 50 μl of 0.5% chicken red blood cell solution to the mixture, allowing the resulting mixture to stand at room temperature and then observing hemagglutination. The HI titer was expressed by the maximum dilution number of the serum in which inhibition of hemagglutination was observed.

The effect of the vaccine is shown in Table 2. The chicken inoculated with the vaccine of the present invention showed take pox as in ordinary vaccine but developed no pox forming by the challenge of the virulent chicken pox virus (3 weeks after the inoculation). Furthermore, the neutralizing antibody to Newcastle disease increased; while HI antibody did not increase, 100% of the chicken survived against the challenge with virulent NDV (2 weeks after the inoculation).

From these results, it is shown that the vaccine of the present invention can immunize chicken pox and Newcastle disease at the same time.

11

Table 2

| Group | Number of Chicken | State of Pox Formation | ND-HI Titer 2 weeks | 3 weeks | Neutralizing Antibody Titer* | Challenge** FPV | NDV |
|---|---|---|---|---|---|---|---|
| Vaccine group: | | | | | | | |
| | 1 | Pox forming | <1:2 | <1:2 | | – | – |
| | 2 | " | <1:2 | <1:2 | | – | – |
| | 3 | " | <1:2 | <1:2 | 1:160 | – | – |
| | 4 | " | <1:2 | <1:2 | | – | – |
| | 5 | " | <1:2 | <1:2 | | – | – |
| Intact group: (control) | | | | | | | |
| | 1 | No pox forming | <1:2 | <1:2 | | + | + |
| | 2 | " | <1:2 | <1:2 | | + | + |
| | 3 | " | <1:2 | <1:2 | <1:2 | + | + |
| | 4 | " | <1:2 | <1:2 | | + | + |
| | 5 | " | <1:2 | <1:2 | | + | + |

* Sera collected from 5 chicken were pooled and measured.

** FPV (virulent strain of fowlpox virus)

+: Pox forming was noted 3 weeks after.

–: No pox forming was noted 3 weeks after.

ND (virulent NDV Sato strain)

+: Pox forming was noted 2 weeks after.

–: No pox forming was noted 2 weeks after.

Claims

1. A recombinant Avipoxvirus having inserted the whole or a part of cDNA coding for Newcastle disease virus-derived fused protein in a genome region non-essential to proliferation of Avipoxvirus.

2. A recombinant Avipoxvirus as claimed in claim 1, wherein said cDNA is under control of a promoter.

3. A recombinant Avipoxvirus as claimed in claim 1 or 2, wherein said cDNA encodes an amino acid sequence shown in Figure 3 or polypeptide having substantially the same function as said amino acid sequence.

4. A recombinant Avipoxvirus as claimed in claim 1 or 2, wherein said cDNA has a nucleotide sequence shown in Figure 3 or nucleotide sequence having substantially the same function as said nucleotide sequence.

5. A vaccine for fowl which comprises a recombinant Avipoxvirus claimed as in claim 1, 2, 3 or 4.

6. A vaccine for fowl capable of imparting to fowl immunity against fowlpoxvirus and Newcastle disease virus, comprising a recombinant Avipoxvirus claimed in claim 1 or 2.

7. A recombinant Avipoxvirus according to claim 1 or claim 2, wherein the cDNA has a sequence substantially homologous to all or part of the sequence shown in Fig. 3, or is capable of annealing with all or part of said sequence or its complementary sequence.

8. A process of preparing a recombinant Avipoxvirus according to any one of claims 1 to 4 or 7, comprising inserting said cDNA sequence in an Avipoxvirus in a genome region non-essential to proliferation of Avipoxvirus.

9. A process for preparing a vaccine capable of imparting immunity against fowlpoxvirus and Newcastle disease virus which comprises a process of preparing a recombinant Avipoxvirus according to claim 8.

# F I G. I

E: EcoRI, H: HindⅢ, B: BamHI, G: BgℓⅡ, S: SmaI

K: KpnI, Ava: AvaⅡ, Sac: SacI, X: XbaI

# F I G. 2

VECTOR DNA · AVIPOXVIRUS DNA FRAGMENT · NDV-F GENE · [P] 7.5k PROMOTER

E: EcoRI,  V: EcoRV,  H: HindⅢ,  B: BamHI,  S: SmaI,  X: XbaI

# F I G. 3-1

```
                                        27                                            54
GTA GAA GAT TCT GGA TCC CGG TTG GCG CCT TCA AGG TGC AAG ATG GGC TCC AGA
                                                        Met Gly Ser Arg


                                        81                                           108
TCT TCT ACC AGG ATC CCA GTA CCT CTG ATG CTG ACC GTC CGA ATC ATG TTG GCA
Ser Ser Thr Arg Ile Pro Val Pro Leu Met Leu Thr Val Arg Ile Met Leu Ala


                                       135                                           162
CTG AGT TGC GTC TGT CCG ACC AGC TCC CTT GAT GGC AGG CCT CTT GCA GCT GCA
Leu Ser Cys Val Cys Pro Thr Ser Ser Leu Asp Gly Arg Pro Leu Ala Ala Ala


                                       189                                           216
GGG ATT GTG GTA ACA GGA GAC AAA GCA GTC AAC ATA TAC ACC TCA TCT CAG ACA
Gly Ile Val Val Thr Gly Asp Lys Ala Val Asn Ile Tyr Thr Ser Ser Gln Thr


                                       243                                           270
GGG TCA ATC ATA ATC AAG TTA CTC CCA AAT ATG CCC AAG GAT AAA GAG GCG TGT
Gly Ser Ile Ile Ile Lys Leu Leu Pro Asn Met Pro Lys Asp Lys Glu Ala Cys


                                       297                                           324
GCA AAA GCC CCA TTG GAA GCA TAC AAC AGG ACA TTG ACT ACT TTG CTC ACC CCC
Ala Lys Ala Pro Leu Glu Ala Tyr Asn Arg Thr Leu Thr Thr Leu Leu Thr Pro


                                       351                                           378
CTT GGT GAT TCT ATC CGT AGG ATA CAA GAG TCT GTG ACC ACA TCC GGA GGA GGG
Leu Gly Asp Ser Ile Arg Arg Ile Gln Glu Ser Val Thr Thr Ser Gly Gly Gly


                                       405                                           432
AAA CAG GGA CGT CTT ATA GGC GCC ATT ATC GGT GGT GTA GCT CTC GGG GTT GCA
Lys Gln Gly Arg Leu Ile Gly Ala Ile Ile Gly Gly Val Ala Leu Gly Val Ala


                                       459                                           486
ACC GCT GCA CAG ATA ACA GCA GCC TCG GCT CTG ATA CAA GCC AAT CAA AAT GCT
Thr Ala Ala Gln Ile Thr Ala Ala Ser Ala Leu Ile Gln Ala Asn Gln Asn Ala


                                       513                                           540
GCC AAC ATC CTC CGG CTC AAA GAG AGC ATT GCT GCA ACC AAT GAG GCT GTG CAC
Ala Asn Ile Leu Arg Leu Lys Glu Ser Ile Ala Ala Thr Asn Glu Ala Val His
```

# FIG. 3-2

```
                                    567                                    594
GAG GTC ACT GAC GGA TTA TCA CAA CTA GCA GTG GCA GTT GGG AAG ATG CAG CAA
Glu Val Thr Asp Gly Leu Ser Gln Leu Ala Val Ala Val Gly Lys Met Gln Gln


                                    621                                    648
TTT GTT AAT GAC CAG TTT AAT AAA ACA GCT CAG GAA TTG GAC TGT ATA AAA ATT
Phe Val Asn Asp Gln Phe Asn Lys Thr Ala Gln Glu Leu Asp Cys Ile Lys Ile


                                    675.                                   702
ACA CAG CAG GTT GGT GTA GAA CTC AAC CTG TAC CTA ACT GAA TTG ACT ACA GTA
Thr Gln Gln Val Gly Val Glu Leu Asn Leu Tyr Leu Thr Glu Leu Thr Thr Val


                                    729                                    756
TTC GGG CCA CAA ATC ACT TCC CCT GCC TTA ACT CAG CTG ACT ATC CAG GCG CTT
Phe Gly Pro Gln Ile Thr Ser Pro Ala Leu Thr Gln Leu Thr Ile Gln Ala Leu


                                    783                                    810
TAC AAT CTA GCT GGT GGG AAT ATG GAT TAC TTG TTG ACT AAG TTA GGT GTA GGA
Tyr Asn Leu Ala Gly Gly Asn Met Asp Tyr Leu Leu Thr Lys Leu Gly Val Gly


                                    837                                    864
AAC AAC CAA CTC AGC TCA TTA ATT GGT AGT GGC CTG ATT ACC GGC AAC CCT ATC
Asn Asn Gln Leu Ser Ser Leu Ile Gly Ser Gly Leu Ile Thr Gly Asn Pro Ile


                                    891                                    918
CTG TAC GAC TCA CAG ACT CAA CTC TTG GGT ATA CAG GTC ACC CTA CCC TCA GTC
Leu Tyr Asp Ser Gln Thr Gln Leu Leu Gly Ile Gln Val Thr Leu Pro Ser Val


                                    945                                    972
GGG AAT CTA AAT AAT ATG CGT GCC ACC TAC CTG GAA ACC TTG TCT GTA AGT ACA
Gly Asn Leu Asn Asn Met Arg Ala Thr Tyr Leu Glu Thr Leu Ser Val Ser Thr


                                    999                                    1026
ACC AAA GGA TTT GCC TCA GCA CTT GTC CCA AAA GTA GTG ACA CAG GTT GGT TCC
Thr Lys Gly Phe Ala Ser Ala Leu Val Pro Lys Val Val Thr Gln Val Gly Ser


                                    1053                                   1080
GTG ATA GAA GAG CTT GAC ACC TCG TAC TGT ATC GAG ACC GAT TTG GAC CTA TAT
Val Ile Glu Glu Leu Asp Thr Ser Tyr Cys Ile Glu Thr Asp Leu Asp Leu Tyr


                                    1107                                   1134
TGT ACA AGA ATA GTG ACA TTC CCT ATG TCT CCT GGT ATT TAT TCC TGT TTG AGT
Cys Thr Arg Ile Val Thr Phe Pro Met Ser Pro Gly Ile Tyr Ser Cys Leu Ser
```

# FIG. 3-3

```
                              1161                                    1188
GGC AAT ACA TCT GCT TGC ATG TAT TCA AAG ACT GAA GGC GCA CTC ACT ACG CCG
Gly Asn Thr Ser Ala Cys Met Tyr Ser Lys Thr Glu Gly Ala Leu Thr Thr Pro


                              1215                                    1242
TAT ATG ACC CTC AAA GGC TCA GTT ATT GCC AAC TGT AAG ATG ACA ACA TGT AGA
Tyr Met Thr Leu Lys Gly Ser Val Ile Ala Asn Cys Lys Met Thr Thr Cys Arg


                              1269                                    1296
TGT GCA GAC CCC CCG GGT ATC ATA TCG CAG AAT TAT GGA GAA GCT GTG TCT CTA
Cys Ala Asp Pro Pro Gly Ile Ile Ser Gln Asn Tyr Gly Glu Ala Val Ser Leu


                              1323                                    1350
ATA GAT AGG CAA TCA TGC AAT ATC TTA TCC TTA GAC GGG ATA ACT TTG AGG CTC
Ile Asp Arg Gln Ser Cys Asn Ile Leu Ser Leu Asp Gly Ile Thr Leu Arg Leu


                              1377                                    1404
AGT GGG GAA TTT GAT GCA ACT TAT CAA AAG AAT ATC TCA ATA CAA GAT TCT CAA
Ser Gly Glu Phe Asp Ala Thr Tyr Gln Lys Asn Ile Ser Ile Gln Asp Ser Gln


                              1431                                    1458
GTA ATA GTT ACA GGC AAT CTT GAC ATC TCG ACT GAG CTT GGG AAT GTC AAC AAC
Val Ile Val Thr Gly Asn Leu Asp Ile Ser Thr Glu Leu Gly Asn Val Asn Asn


                              1485                                    1512
TCG ATA AGT AAT GCT TTG GAT AAG TTA GAG GAA AGC AAC AGC AAA CTA GAC AAG
Ser Ile Ser Asn Ala Leu Asp Lys Leu Glu Glu Ser Asn Ser Lys Leu Asp Lys


                              1539                                    1566
GTC AAT GTT AAA CTG ACC AGC ACA TCC GCT CTT ATT ACC TAT ATC TTT TTA ACT
Val Asn Val Lys Leu Thr Ser Thr Ser Ala Leu Ile Thr Tyr Ile Phe Leu Thr


                              1593                                    1620
GTC ATA TCT CTT GTA TGT GGT ATA CTT AGC CTG GTT CTA GCA TGC TAC CTG ATG
Val Ile Ser Leu Val Cys Gly Ile Leu Ser Leu Val Leu Ala Cys Tyr Leu Met


                              1647                                    1674
TAC AAG CAA AAG GCG CAA CAG AAG ACC TTG TTG TGG CTT GGG AAT AAT ACC CTA
Tyr Lys Gln Lys Ala Gln Gln Lys Thr Leu Leu Trp Leu Gly Asn Asn Thr Leu


                              1701
GAC CAG ATG AGG GCC ACT ACA AAA ATG TGA
Asp Gln Met Arg Ala Thr Thr Lys Met
```

EP 0 404 576 A2

# FIG. 4

(a)

E · · · V X · · · Hc · · · · · P Hc H

0 · 1 · 2 · 3 · 4 · 5 Kbp

(b)

B X · V · H · · · V V EᴴHcᴴ B

0 · 1 · 2 · 3 · 4 Kbp

(c)

B C · K X · · V · XᴴHc V · · H B

0 · 1 · 2 · 3 3.3 Kbp

(d)

H G Hc Hc)C · · · · · P · G · · · Xb Hc Hp Xb · H

0 · 1 · 2 · 3 · 4 · 5 5.2 Kbp

E:EcoRI  P:PstI  G:BgℓII
X:XbaI  B:BamHI
V:EcoRV  H:HindIII
Hc:HincII  C:CℓaI
K:KpnI  Xb:XbaI  Hp:HpaI

(e)

V Hp V X · · H

0 · 1 · 1.8 Kbp

FIG. 5

AVIPOXVIRUS DNA

CA. 4.0Kbp

BamHI

B / B CA. 4.0 Kbp FRAGMENT

EcoRI HindⅢ

B pNZ160 B

pUC9

BamHI  LIGASE
ALKALINEPHOSPHATASE

H E CA. 5.0Kbp FRAGMENT

CA. 5.0Kbp

pUC18

EcoRI HindⅢ
ALKALINEPHOSPHATASE

LIGASE

H pNZ180H E

EcoRI

EcoRI
ALKALINEPHOSPHATASE

LIGASE

V V H

pNZ136

E

CA. 1.8Kbp FRAGMENT
HindⅢ DNA POLYMERASE
EcoRV PARTIAL LIGASE

CA. 1.8 Kbp
XbaI

HpaI

V

(V) pNZ136S (H)

E E CA. 7.3Kbp FRAGMENT

MULTI CLONING SITE

H
E

pUC18

HindⅢ, EcoRI

E: EcoRI    V: EcoRV    H: HindⅢ    B: BamHI

EP 0 404 576 A2

# F I G. 6

—VECTOR DNA  ⊡7.5K PROMOTER  ▭NDV-F GENE

E:EcoRI,  H:HindⅢ,  B:BamHI,  HpⅡ:HpaⅡ,  S:SmaI

# F I G. 7

VECTOR DNA    AVIPOXVIRUS DNA FRAGMENT    β β-gal DNA    P 7.5K PROMOTER

E:EcoRI   V:EcoRV   H:HindIII   B:BamHI   S:SmaI   X:XbaI

EP 0 404 576 A2

# F I G. 8

pNZI36S

V   X

i) EcoRV
ii) EcoRI LINKER, LIGASE
iii) EcoRI, XbaI

i) EcoRV
ii) HindⅢ LINKER, LIGASE
iii) HindⅢ, XbaI

H   E

pUCI8

HindⅢ
EcoRI

Sp Saℓ B K   E
H   P   X  S Sac
51 bp

H   X

E   X

LIGASE

MULTICLONING
SITE

H B E

(V)   pNZI36SL   (H)

E : EcoRI
B : BamHI
H : HindⅢ
V : EcoRV
X : XbaI
Saℓ : Saℓ I
S : SmaI
Sac : SacI
Sp : SphI
K : KpnI

AVIPOXVIRUS
DNA FRAGMENT

—— VECTOR DNA